# EUROPÄISCHE PATENTANMELDUNG

(11) **EP 1 927 844 A1**
(43) Veröffentlichungstag der Anmeldung: **04.06.2008**
(21) Anmeldenummer: 06024587.5
(22) Anmeldetag: 28.11.2006
(51) Int. Cl.: G01N 13/02, G01N 33/15

(54) **Prüfung von Benetzungseigenschaften**

(71) Anmelder: Bayer CropScience AG, 40789 Monheim (DE)
(72) Erfinder: Süssmann, Rainer, D-65549 Limburg (DE); Baur, Peter, D-86938 Schondorf (DE); Franz, Jörg, D-65594 Runkel (DE)

(57) **Zusammenfassung**

Ein Träger mit einem speziellen Oberflächenprofil, welches die Benetzungseigenschaften von natürlichen Oberflächen simuliert, wird in einem speziellen Verfahren zur Prüfung von Benetzungseigenschaften verwendet. Träger und Verfahren können in den Bereichen Pharma, Tiergesundheit, Kosmetik und Pflanzenschutz eingesetzt werden.
Das Oberflächenprofil weist Rillen mit im Mittel eine Rillentiefe (MRT) von 2-10 μm und im Mittel eine Rillenbreite von (MRB) 2-10 μm auf.

## Beschreibung

Die vorliegende Erfindung betrifft das Gebiet der Bestimmung der Benetzbarkeit von Festkörperoberflächen. Insbesondere betrifft die Erfindung einen Träger und ein Verfahren mit welchen Benetzungseigenschaften von Flüssigkeiten, bevorzugt Lösungen (Formulierungen) von Wirkstoffen, insbesondere agrochemischen Wirkstoffen, auf einfache Art und Weise geprüft werden können.

Verfahren zur Bestimmung der Benetzung und Benetzbarkeit sind bekannt. Im Gebiet der Grenzflächenphysik wird die Interaktion flüssiger und fester Stoffe durch die Betrachtung der Verhältnisse der Ober- und Grenzflächenspannung der beteiligten Phasen und Phasengrenzen beschrieben.

Die Verhältnisse werden anschaulich durch die Young-Gleichung [Young, T., Phil. Trans. Roy. Soc. (1805), 95, S. 65 und Girifalco, L. A., Good, R. J., J. Phys. Chem. (1957), 61, S. 904; ibid. (1960), 64, S. 561] beschrieben: Ein Flüssigkeitstropfen auf einem ideal glatten Festkörper bildet an seinem Rand eine Grenzlinie, an der sich die drei Phasen treffen. Die Tropfenform, als sich einstellende Eigenschaft, gibt die herrschenden Verhältnisse der Grenz- und Oberflächenspannungen - d.h. Energien - wieder.

Durch Anlegen einer Tangente an den Punkt, wo der aufgesetzte Flüssigkeitstropfen die Festkörperoberfläche und die Umgebungsphase (meist Luft) berührt, wird der Kontaktwinkel ermittelt. Der Kontaktwinkel Q wird auch Rand-, Young- oder Benetzungswinkel genannt und er kann Werte in den Grenzen 0° < Q < 180° annehmen. Man sagt, eine Flüssigkeit wirkt benetzend bzw. ein Festkörper ist durch sie benetzbar oder ist benetzt, wenn der Kontaktwinkel Q < 90° beträgt. Die Güte der Benetzung ist im Kontaktwinkel indirekt proportional (z.B. der Kontaktwinkel von Quecksilber auf Glas beträgt etwa 138°, da Glas von Quecksilber nicht benetzt wird; der Fall Q = 0° = totale Benetzung bzw. Spreitung oder Q = 180° = totale Nicht-Benetzung tritt in der Wirklichkeit allerdings nicht auf).

Der Kontaktwinkel zwischen einer Flüssigkeit und einem Festkörper, als Maß für die energetische Wechselwirkung zwischen dem Festkörper und der Flüssigkeit, wird meist nach der Methode des liegenden Tropfens bestimmt. Eine weitere Möglichkeit ist die 'captive-bubble'-Methode, die sich für Materialien eignet, die feucht gehalten werden müssen, wie etwa biologische Proben oder manche Membranen. In beiden Fällen wird an den Dreiphasenpunkt eine Tangente angelegt, deren Winkel zu der Festkörperoberfläche als Kontaktwinkel definiert ist. Alternativ kann der Kontaktwinkel auch aus der Tropfenkontur durch verschiedene rechnerische Verfahren (z.B. über Young-Laplace-Gleichung) bestimmt werden.

Die Messung der Kontaktwinkel erfolgt über spezifische Apparaturen, wie Kontaktwinkel-Messsysteme (z.B. von der Fa. Krüss GmbH, Deutschland; www.kruss.de).

Diese Verfahren liefern genaue Messergebnisse bezüglich der Benetzungseigenschaften. Jedoch sind die Kontaktwinkel-Meßsysteme bei Bestimmungen der Benetzungseigenschaften auf den erfindungsgemäßen und natürlichen Oberflächen, wie Pflanzen- und Insektenkutikula sowie Haut, nicht oder nur bedingt anwendbar, da oft eine zu schnelle Benetzung (Spreitung) stattfindet. Bei biologischen Oberflächen kann andererseits durch Penetration der grenzflächenaktiven Komponenten und/oder weiterer Komponenten das Ergebnis verfälscht werden. Vielmehr werden in diesen Fällen kostengünstige und einfache, vom Laien durchführbare Schnelltests, mit denen Unterschiede in den Benetzungseigenschaften von Flüssigkeiten festgestellt werden können, vorgezogen.

Aufgabe der vorliegenden Erfindung bestand deshalb darin, einen solchen Schnelltest bereitzustellen, mit dem ein unterschiedliches Benetzungsverhalten der zu prüfenden Flüssigkeiten gut erkannt werden kann.

Diese Aufgabe wird gelöst durch ein Verfahren bei dem spezifisch aufgeraute Trägeroberflächen aus speziellen Materialen verwendet werden.

Gegenstand der Erfindung ist ein Träger zur Bestimmung von Benetzungseigenschaften, dadurch gekennzeichnet, dass sein Oberflächenprofil im Mittel eine Rillentiefe (MRT) von 2 -10 µm und im Mittel eine Rillenbreite von (MRB) 2 - 10 µm aufweist.

Bevorzugte Träger werden ausgewählt aus der Gruppe der Kunststoffe, besonders bevorzugt aus der Gruppe der Thermoplaste und ganz besonders bevorzugt aus der Gruppe der Polystyrole und Polyvinylchloride.

Weiterer Gegenstand der Erfindung ist ein Verfahren zur Bestimmung der Benetzungseigenschaften von Flüssigkeiten, bei dem die Flüssigkeit auf einem erfindungsgemäßen Träger aufgebracht wird, dessen Oberflächenprofil und Beschaffenheit (Material) die oben genannten Spezifikationen aufweist.

Form und Größe des Trägers stellen, bei Einhaltung des Mindestmaßes (2,3 cm im Kreisdurchschnitt einer speziell behandelten Fläche) keine Limitation dar und können daher entsprechend beliebig gewählt werden. Die bevorzugte Form der Trägerplatte hat eine Fläche von 8,5 x 12,5 cm mit insgesamt 12 Kreissegmenten.

Die zur Bestimmung ihrer Benetzungseigenschaften verwendeten Flüssigkeiten müssen nur über eine ausreichende Viskosität verfügen, um im erfindungsgemäßen Verfahren eingesetzt werden zu können. Ihre Zusammensetzung stellt für die Durchführung des Verfahrens keine Limitation dar und kann daher beliebig sein.

Bevorzugt sind Flüssigkeiten, die als Lösungen Wirkstoffe enthalten. Diese Wirkstoffe können aus den Bereichen Pharma, Tiergesundheit, Kosmetik und Pflanzenschutz stammen, bevorzugt aus dem Bereich Pflanzenschutz, ganz besonders bevorzugt aus dem Bereich Pflanzenschutz-Herbizide. Die Wirkstoffe können entweder unformuliert oder formuliert in den Lösungen angesetzt werden.

Bevorzugt ist die Verwendung formulierter Wirkstoffe für den Ansatz der Lösungen.

Zur Aufbringung der Flüssigkeit auf dem Träger können unterschiedliche Techniken verwendet werden. Bevorzugt sind hierbei Tropf- und Pipettiervorgänge, ganz besonders bevorzugt Pipettiervorgänge.

Die Bestimmung der Benetzungseigenschaften erfolgt entweder anhand der Spreitung auf einer festgelegten Fläche aufgrund der gemessenen Zeit oder anhand einer festgelegten Zeit aufgrund der gemessenen Fläche, die die Flüssigkeit benetzt hat. Bevorzugt ist die Bestimmung aufgrund der festgelegten Fläche.

Zur Unterstützung der Bestimmung der Benetzungseigenschaften können Farb- und/oder Fluoreszenz-Stoffe oder ähnliches den zu messenden Flüssigkeiten beigemischt werden.

Eine besondere Ausführungsform der Erfindung ist ein Test-Kit bestehend aus einer Grundplatte (Träger), vorzugsweise 8,5 x 12,5 cm, mit kreisförmig aufgerauten Segmenten, vorzugsweise 12, mit einem Durchmesser von je 2-3 cm, und einer Deckelplatte, wobei der Träger dadurch gekennzeichnet ist, dass er aus Material aus der Gruppe der Thermoplaste besteht und sein Oberflächenprofil im Mittel eine Rillentiefe (MRT) von 2-10 µm und im Mittel eine Rillenbreite (MRB) von 2-10 µm aufweist.

Die Ermittlung der Benetzung von oben beschriebenen Flüssigkeiten erfolgt mit Hilfe des Test-Kits nach Applikation eines Tropfens in beschriebener Weise und anschließender optischer Beurteilung seines Fließverhaltens.

Das oben beschriebene Test-Kit wird u.a. unter der Bezeichnung 'ODesi-Test-Kit' engesetzt.

Ein weiterer Gegenstand der Erfindung ist ein Verfahren zur Herstellung von Trägern mit dem erfindungsgemäßen Oberflächenprofil, dadurch gekennzeichnet, dass das Oberflächenprofil, durch mechanische und/oder chemische Aufrauhung erzielt wird. Die mechanische Aufrauhung erfolgt bevorzugt durch Schleifpapier, Schleifbürste und/oder Sandstrahlung. Besonders bevorzugt ist hierbei die Verwendung einer rotierenden Schleifbürste mit folgender Spezifikation:
- Drahtbürste mit ca. 300-500 Borsten auf 2 cm²,
- Borstendurchmesser 130 ± 10 µm,
- 20 000 Umdrehungen pro Minute,
- 200 -300 g Anpressdruck,
- Dauer: 2-3 Minuten.

Weiterhin Gegenstand der Erfindung sind die Verwendung des erfindungsgemäßen Trägers und die Verwendung des erfindungsgemäßen Verfahrens zur Bestimmung von Benetzungseigenschaften von Flüssigkeiten.

Die Erfindung wird durch die Beispiele näher erläutert ohne sie darauf zu beschränken.

### Beispiele:

### 1. Vergleichsversuche zur Ermittlung des Oberflächenprofils eines Trägers zur Simulation von Pflanzenblattoberflächen

### 1.1 Versuchsdurchführung

Unterschiedliches Trägermaterial wurde kreisrund ausgeschnitten (Durchmesser 5 cm). Nach einer Behandlung der Oberfläche zur Erzielung unterschiedlicher Oberflächenprofile wurde mit einer 0,5 -10 µl Eppendorfpipette jeweils ein Tropfen von 8 µL auf die verschiedenen Träger aufgebracht und das Spreitverhalten in Abhängigkeit von der Zeit gemessen.

Die für die Versuche verwendete Lösung bestand aus einer Spritzbrühe, hergestellt aus der Handelsformulierung des Produktes 'Atlantis OD' (zugelassen in Deutschland unter der Nr. 5938-00, Vertrieb durch Bayer CropScience).

### 1.2 Tabelle: Ergebnisse (MRT = Mittlere Rillentiefe in µm; MRB = Mittlere Rillenbreite in µm)

| **Träger-Material** | **Träger-Herstellung** | **Spreitung Ø mm** | **Zeit in Sekunden** |
|---|---|---|---|
| Kunststoff/ Polystyrol | Keine Oberflächenbehandlung | 8 | 180 |
| | (1)MRT2-10 MRB2-10 | 25 | 30 |
| | (2) MRT 50 MRB 50 - 150 | 35 | 30 (400er) |
| | (2) MRT 50 MRB 50 - 150 | 35 | 15 (60er, 150er) |
| Glas | Keine Rillenstrukturen erkennbar | 7 | 180 |
| | (2) MRT - n.b. MRB - n.b. | 10 | 60 (400er) |
| | (2) MRT - n.b. MRB - n.b. | 10 | 20 (60er, 150er) |
| Teflon/ PTFE | Es wurden keine Rillenstrukturen bestimmt | 3 | 180 |
| | (1) MRT - n.b. MRB - n.b. | 0 | 180 |
| | (2) MRT - n.b. MRB - n.b. | 0 | 180 |

| | | | |
|---|---|---|---|
| Anmerkungen: (1) Behandlung mit rotierender Drahtbürste (gemäß Spezifikationen im Text); (2) Behandlung mit Schleifpapier (Körnung in Klammern, wie 60er, 150er, 400er); n.b. = Vorversuche, MRT und MRB nicht bestimmt bzw. nicht bestimmbar. | | | |

### 1.3 Tabelle: Ergebnisse (MRT = Mittlere Rillentiefe in µm; MRB = Mittlere Rillenbreite µm)

| **Durchmesser der Spreitung (mm)** | | **10** | **20** | **25** | **28** | **35** |
|---|---|---|---|---|---|---|
| **Träger-Material** | **Träger-Herstellung** | | | | | |
| Pflanzenblatt *) | Keine | < 60 sek. | 60 sek. | 120 sek. | 180 sek. | > 180 sek. |
| Kunststoff Polystyrol | (1) MRT 2 - 10 MRB 2 - 10 | | | 30 sek. | | |
| | (2) MRT 50 MRB 50-150 | | | | | 30 sek. |
| Glas | (2) MRT - n.b. MRB - n.b. | 60 sek. (400er) | | | | |

| | | | | | | |
|---|---|---|---|---|---|---|
| Anmerkungen: (1) Behandlung mit rotierender Drahtbürste (gemäß Spezifikationen im Text); (2) Behandlung mit Schleifpapier (Körnung in Klammem); n.b. = nicht bestimmt bzw. nicht bestimmbar; sek. = Sekunden; *) Blatt der Pflanze Schefflera arboricola. | | | | | | |

### 1.4 Diskussion der Ergebnisse

Die Ergebnisse in der Tabelle 1.2 zeigen den Einfluss verschiedener Oberflächenprofile, die durch verschiedene Verfahren der Träger-Herstellung auf verschiedenartigen Materialien erzielt wurden. So erwies sich das Oberflächenprofil aus Teflon und Glas als ungeeignet für das Verfahren, da keine oder eine zu geringe Spreitung stattfand.

Tabelle 1.3 zeigt die Ergebnisse der Bestimmung von Benetzungseigenschaften auf den erfindungsgemäßen Trägern im direkten Vergleich mit einer natürlichen Oberfläche. In diesem Beispiel wurde versucht ein geeignetes Oberflächenprofil zur Simulation der Spreitung auf einem Pflanzenblatt zu finden. Für diesen Fall scheint das Oberflächenprofil, gekennzeichnet durch eine MRT von 2 -10 µm und eine MRB 2 - 10 µm am besten geeignet zu sein.

Für andere natürliche Oberflächen können andere erfindungsgemäße Träger mit entsprechenden Oberflächenprofilen, gekennzeichnet jeweils durch spezielle MRT- und MRB-Werte, notwendig sein. Diese Werte können dann wie im vorliegenden Beispiel (analog Tabelle 1.2) ermittelt werden. Dabei ist es unerheblich mit welchen Verfahren der Träger-Herstellung das zur Bestimmung von Benetzungseigenschaften hierfür notwendige Oberflächenprofil erzielt wird.

## Patentansprüche

1. Träger zur Bestimmung von Benetzungseigenschaften, **dadurch gekennzeichnet, dass** sein Oberflächenprofil im Mittel eine Rillentiefe (MRT) von 2 - 10 µm und im Mittel eine Rillenbreite von (MRB) 2 - 10 µm aufweist.

2. Träger gemäß Anspruch 1, bestehend aus Material aus der Gruppe Kunststoff, bevorzugt aus der Gruppe der Thermoplaste.

3. Verfahren zur Bestimmung der Benetzungseigenschaften von Flüssigkeiten, bei dem die Flüssigkeit auf einem speziellen Träger gemäß einem oder mehreren der Ansprüche 1 oder 2 aufgebracht wird.

4. Verfahren gemäß Anspruch 3, wobei es sich bei den Flüssigkeiten um Lösungen von Wirkstoffen, vorzugsweise formuliert, handelt.

5. Verfahren gemäß Anspruch 3, wobei die Flüssigkeiten aufpipettiert werden.

6. Test-Kit bestehend aus einer Grundplatte (Träger), vorzugsweise 8,5 x 12,5 cm, mit kreisförmig aufgerauten Segmenten, vorzugsweise 12, mit einem Durchmesser von je 2-3 cm, und einer Deckelplatte, wobei der Träger **dadurch gekennzeichnet ist, dass** er aus Material aus der Gruppe der Thermoplaste besteht und sein Oberflächenprofil im Mittel eine Rillentiefe (MRT) von 2-10 µm und im Mittel eine Rillenbreite (MRB) von 2-10 µm aufweist.

7. Verfahren zur Herstellung eines Trägers gemäß einem oder mehreren der Ansprüche 1 oder 2, wobei das Oberflächenprofil, durch mechanische Aufrauhung erzielt wird, bevorzugt durch Schleifpapier, Schleifbürste und/oder Sandstrahlung.

8. Verwendung des Trägers gemäß einem oder mehreren der Ansprüche 1 oder 2 zur Bestimmung von Benetzungseigenschaften von Flüssigkeiten.

9. Verwendung des Verfahrens gemäß einem oder mehreren der Ansprüche 3 bis 6 zur Bestimmung von Benetzungseigenschaften von Flüssigkeiten.
